# FASCICULE DE BREVET EUROPEEN

(11) **EP 1 877 142 B1**
(45) Date de publication et mention de la délivrance du brevet: **25.05.2011**
(21) Numéro de dépôt: 06755459.2
(22) Date de dépôt: 27.04.2006
(51) Int. Cl.: A61Q 5/10, A61K 8/49, A61K 8/40, A61K 8/34, A61K 8/37

(54) **COMPOSITION TINCTORIALE COSMETIQUE COMPRENANT UN COLORANT HYDROPHOBE ET UN DERIVE DE PROPYLENE GLYCOL**
KOSMETISCHE FÄRBEZUSAMMENSETZUNG MIT WASSERABWEISENDEM FARBSTOFF UND EINEM PROPYLENGLYKOLDERIVAT
COSMETIC DYEING COMPOSITION COMPRISING A HYDROPHOBIC DYE AND A PROPYLENE GLYCOL DERIVATIVE

(30) Priorité: 27.04.2005 FR 0551085; 04.05.2005 US 677348 P
(43) Date de publication de la demande: 16.01.2008
(73) Titulaire: L'Oréal, 75008 Paris (FR)
(72) Inventeur: DE BONI, Maxime, F-75020 Paris (FR); LAGRANGE, Alain, F-77700 Coupvray (FR)
(74) Mandataire: Fevrier, Murielle Françoise E.
(86) Numéro de dépôt international: PCT/FR2006/000955
(87) Numéro de publication internationale: WO 2006/114530

(56) Documents cités:
- EP-A- 0 406 887
- EP-A- 0 529 598
- FR-A- 1 269 591
- US-A- 3 578 387

## Description

L'invention a pour objet une composition de coloration comprenant dans un milieu approprié un colorant hydrophobe particulier et un ou plusieurs dérivés de propylène glycol particuliers. L'invention a aussi pour objet l'utilisation de cette composition pour la teinture des fibres kératiniques ainsi que le procédé de teinture mettant en oeuvre cette composition.

Depuis longtemps, on cherche à modifier la couleur des cheveux et en particulier à masquer les cheveux blancs. Pour ce faire, plusieurs technologies ont été développées.

Il est connu de teindre les matières kératiniques et en particulier les cheveux humains, avec des compositions de teinture contenant des colorants directs. Les colorants classiques qui sont utilisés sont en particulier des colorants du type nitrés benzèniques, anthraquinoniques, nitropyridiniques, azoïques, azoïques, xanthéniques, acridiniques, aziniques, triarylméthane ou des colorants naturels. Ces colorants peuvent être non ioniques, anioniques, cationiques ou amphotères.

Ces colorants qui sont des molécules colorées et colorantes ayant une affinité pour les fibres kératiniques sont appliqués pendant le temps nécessaire à l'obtention de la coloration désirée, puis rincés.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, il est connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs. Les colorations qui en résultent sont permanentes, puissantes, et résistantes aux agents extérieurs, notamment à la lumière, aux intempéries, aux lavages, à la transpiration et aux frottements. Cependant, ce type de coloration entraîne une dégradation de la fibre due à l'utilisation d'un agent oxydant.

FR 1 269 591 décrit des compositions de teinture de cheveux améliorées, traitant les cheveux en une étape et susceptibles de produire une grande variété de teinte, avec des nuances prononcées et résistant à la lumière et aux shampooings (ténacité). Les colorants utilisés sont des colorants basiques, par exemple le solvant orange 15. Les compostions mentionnées dans FR 1 296 591 contiennent comme solvants en milieu aqueux, des alcools ou des éthers solubles dans l'eau et en particulier des monoéthyl-, monométhyl- et monobuthyléthers de l'éthylène glycol.

Il existe toujours un besoin de développer de nouvelles compositions de teinture directe pour d'obtenir des nuances variées, en particulier dans des nuances pastels et qui présentent une bonne ténacité, notamment aux agents extérieurs tels que la lumière, le shampooing, la sueur tout en préservant la qualité des fibres kératiniques. En particulier, il existe un besoin de développer des compositions de coloration permettant d'obtenir des colorations présentant une ténacité proche de la coloration par oxydation sans les inconvénients liés à la présence d'un agent oxydant.

Ce but est atteint avec la présente invention qui a pour objet une composition de coloration cosmétique comprenant dans un milieu de coloration cosmétique approprié, au moins un colorant direct hydrophobe dont le logP est supérieur à 2, le milieu approprié contenant au moins 40 % d'eau en poids par rapport au poids total de la composition de coloration, et au moins un dérivé de propylène glycol de formule (I) suivante

R₁ (OC₃H₆)ₙOR₂

dans laquelle R₁ représente l'hydrogène, un radical alkyle en C₁-C₆ ou acyle C₂-C₆, R₂ représente un radical alkyle en C₁-C₆ ou aryle en C₆-C₃₀ et n variant de 1 et 6 inclus, le motif OC₃H₆ pouvant être linéaire ou ramifié.

La composition de l'invention permet d'obtenir des nuances variées et des colorations intenses. De plus, la coloration obtenue permet d'atteindre voire de dépasser la ténacité de la coloration d'oxydation. Ainsi, la coloration obtenue est très résistante aux agents extérieurs, notamment aux lavages répétés.

L'invention a aussi pour objet l'utilisation pour la coloration des fibres kératiniques d'une composition comprenant dans un milieu de coloration cosmétique approprié, au moins un colorant direct hydrophobe dont le logP est supérieur à 2, le milieu approprié contenant de l'eau, et au moins un dérivé de propylène glycol de formule (I) suivante :

R₁(OC₃H₆)ₙOR₂ (I)

dans laquelle R₁ représente l'hydrogène, un radical alkyle en C₁-C₆ ou acyle C₂-C₆, R₂ représente un radical alkyle en C₁-C₆ ou aryle en C₆-C₃₀ et n variant de 1 à 6, le motif OC₃H₆ pouvant être linéaire ou ramifié.

Dans le cadre de l'invention, la valeur du logP représente de façon classique le coefficient de partage du colorant entre l'octanol et l'eau. La valeur du logP peut être calculée selon la méthode décrite dans l'article de Meylan et Howard « Atom / Fragment contribution method for estimating octanol-water partition coefficient », J. Pharm. Sci. 84 :83-92, 1995.. Cette valeur peut aussi être calculée à partir de nombreux logiciels disponibles sur le marché qui détermine la valeur de logP en fonction de la structure d'une molécule. A titre d'exemple, on peut citer le logiciel Epiwin de l'agence de l'environnement des Etats-Unis.

Les colorants directs qui peuvent être utilisés dans la composition de l'invention sont des colorants hydrophobes connus de la technique qui présentent un logP supérieur à 2. A titre d'exemple, on peut citer :

| **Colorant** | **Structure chimique** | **logP** |
|---|---|---|
| Disperse Red 13 | 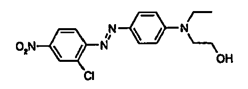 | 5.22 |
| Disperse Green 9 | 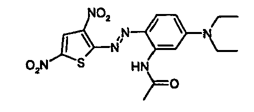 | 4.23 |
| Solvent Black 3 | 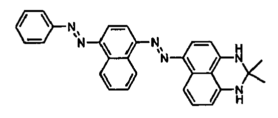 | 7.50 |
| Disperse Blue 148 | 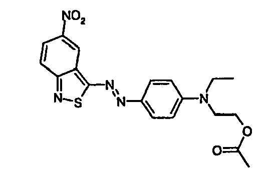 | 4.81 |
| Disperse Violet 63 | 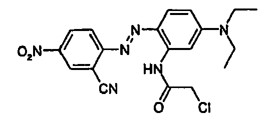 | 5.30 |
| Disperse Blue 60 | 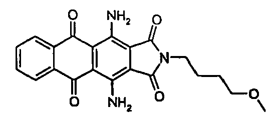 | 3.38 |
| Disperse Blue 14 | 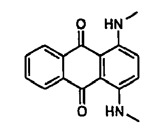 | 4.25 |
| Solvent Orange 15 | 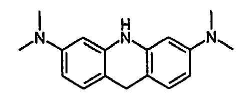 | 3.90 |
| Solvent Orange 7 | 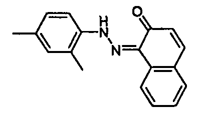 | 4.40 |
| Solvent Blue 14 | 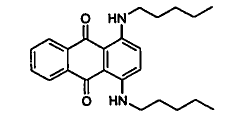 | 8.18 |
| Disperse Yellow 82 | 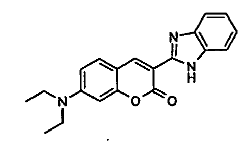 | 3.68 |

Selon un mode de réalisation particulier, un logP du colorant utile dans la composition de l'invention est supérieur à 4.

Le ou les colorants directs présentant un logP supérieur à 2 peuvent être présents dans la composition dans des quantités comprises entre 0,001 à 10 % en poids environ du poids total de la composition.

Dans la composition de l'invention, pour le dérivé de propylène glycol de formule (I), on entend par radical alkyle, les radicaux linéaires ou ramifiés tels que le radical méthyle, éthyle, propyle, isopropyle, isobutyle, tertiobutyle, pentyle, hexyle. A titre de radical aryle, on peut notamment citer les radicaux phényle, benzylz, etc.

Dans la formule (I), le motif OC₃H₆ représente par exemple OCH2CH2CH2, OCH2CH(CH3) ou OCH(CH3)CH2.

A titre de dérivés de propylène glycol de formule (I), on peut citer les propylènes glycols suivants :

| | |
|---|---|
| Dipropylène glycol méthyl éther (Dowanol DPM) | CH₃O(C₃H₆O)₂H |
| Tripropylène glycol méthyl éther (Dowanol TPM) | CH₃O(C₃H₆)₃H |
| Propylène glycol méthyl éther acétate (Dowanol PMA) | CH₃OC₃H₆OCOCH₃ |
| Dipropylène glycol méthyl éther acétate (Dowanol DPMA) | CH₃O(C₃H₆O)₂COCH₃ |
| Propylène glycol n-propyl éther (Dowanol PnP) | C₃H₇OCH₂CH(CH₃)_{OH} |
| Propylène glycol n-butyl éther (Dowanol PnB) | C₄H₉OCH₂CH(CH₃)OH |
| Propylène glycol phényl éther (Dowanol PPh) | C₆H₅OC₃H₆OH |
| Dipropylène glycol n-propyl éther (DPnP) | C₃H₇O[CH₂(CH)CH₃O]₂ H |
| Tripropylène glycol n-propyl éther (TPnP) | C₃H₇O[CH₂(CH)CH₃O]₃ H |
| Dipropylène glycol n-butyl éther (DPnB) | C₄H₉O[CH₂(CH)CH₃O]₂ H |
| Tripropylène glycol n-butyl éther (TPnB) | C₄H₉O[CH₂(CH)CH₃O]₃ H |
| Dipropylène glycol diméthyl éther (DMM) | CH₃O(C₃H₆O)₂CH₃ |

Selon un mode de réalisation particulier, le dérivé de propylène glycol de formule (I) est tel que n varie de 1 à 4 et R₁ représente un radical alkyle en C₁-C₆, de préférence C₁-C₄.

De préférence, si R1 représente l'hydrogène alors n est supérieur à 1 et si n est égal à 1 alors R2 représente un radical alkyle en C2-C6.

La composition de l'invention comprend généralement une quantité de dérivés de propylène glycol de formule (I) comprise entre 0,1 et 40 % en poids du poids total de la composition, de préférence comprise entre 0,5 et 30 %, plus préférentiellement encore de 1 à 20 %.

Selon un mode de réalisation particulier, le milieu approprié à la coloration des fibres kératiniques comprend au moins 70 % d'eau en poids par rapport au poids total de la composition de coloration.

Le milieu de coloration peut par exemple être constitué uniquement par de l'eau ou par un mélange d'eau et d'au moins un solvant organique autre que le dérivé propylène glycol de formule (I). A titre de solvant organique additionnel, on peut par exemple citer les alcanols inférieurs en C₁-C₄, tels que l'éthanol et l'isopropanol ; les polyols et éthers de polyols comme le 2-butoxyéthanol, le monoéthyléther et le monométhyléther du diéthylèneglycol, ainsi que les alcools aromatiques comme l'alcool benzylique ou le phénoxyéthanol, et leurs mélanges.

Pour la coloration des fibres kératiniques humaines, le milieu de coloration est un milieu cosmétique approprié.

La quantité totale de solvant incluant le ou les dérivés de propylène glycol de formule (I) peut varier entre 0,1 et 80 % en poids environ par rapport au poids total de la composition, de préférence entre 0,5 et 50 % en poids environ, de façon encore préférée entre 1 et 30 % en poids.

La composition tinctoriale conforme à l'invention peut en outre contenir des colorants directs différents des colorants directs utiles dans la présente invention. Ces colorants directs additionnels sont par exemple des colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants:
- 1,4-diamino-2-nitrobenzène
- 1-amino-2 nitro-4-β-hydroxyéthylaminobenzène
- 1-amino-2 nitro-4-bis(p-hydroxyéthyl)-aminobenzène
- 1,4-Bis(p-hydroxyéthylamino)-2-nitrobenzène
- 1--hydroxyéthylamino-2-nitro-4-bis-(β-hydroxyéthylamino)-benzène
- 1-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-p-hydroxyéthylamino-5-nitrobenzène
- 1-Méthoxy-2-p-hydroxyéthylamino-5-nitrobenzène
- 1-p-hydroxyéthyloxy-3-méthylamino-4-nitrobenzène
- 1-β-dihydroxypropytoxy-3-méthylamino-4-nitrobenzène
- 1-βhydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772 et EP-714954, FR 2822696, FR 2825702, FR 2825625, FR 2822698, FR 2822693, FR 2822694, FR 2829926, FR 2807650, WO02/078660, WO02/100834, WO 021100369, FR 2844269. dont le contenu fait partie intégrante de l'invention.

Parmi ces composés, on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Acid Yellow 9, Acid Black 1, Basic Red 22, Basic Red 76, Basic Yellow 57, - Basic Brown 16, Acid Yellow 36, Acid Orange 7, Acid Red 33, Acid Red 35, Basic Brown 17, Acid Yellow 23, Acid Orange 24.

On peut aussi citer l'acide 4-hydroxy-3-(2-méthoxyphénylazo)-1-naphtalène sulfonique.

Parmi les colorants directs quinoniques, on peut citer les colorants suivants : Acid Blue 62, Basic Blue 22, Basic Blue 99, ainsi que les composés suivants :
- 1-N-méthylmorpholiniumpropylamino-4-hydroxyanthraquinone
- 1,4-Bis-(β,γ-dihydroxypropylamino)-anthraquinone.

Parmi les colorants aziniques, on peut citer les composés suivants :,Basic Blue 17, Basic Red 2.

Parmi les colorants triarylméthaniques, on peut citer les composés suivants :
- Basic Green 1, Acid blue 9, Basic Violet 3, Basic Violet 14, Basic Blue 7, Acid Violet 49, Basic Blue 26, Acid Blue 7.

Parmi les colorants directs, on peut aussi citer les colorants directs naturels tels que la lawsone, la juglone, l'acide carminique, l'acide kermésique, la purpurogalline, le protocatéchaldéhyde, la spinulosine, l'apigénidine. On peut également utiliser les extraits ou décoctions contenant ces colorants naturels et notamment les cataplasmes ou extraits à base de henné.

Lorsque cette composition comprend d'autres colorants directs que ceux présentant un logP supérieur à 2, la composition peut contenir jusqu'à 20 % de colorants directs. Selon ce mode particulier de réalisation, la composition de l'invention comprend une quantité totale de colorants directs comprise entre 0,001 à 15% en poids environ.

La composition de la présente invention peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Pour la teinture des fibres kératiniques humaines, le milieu de coloration est un milieu cosmétique.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le pH de la composition tinctoriale conforme à l'invention est généralement compris entre 2 et 12 environ, et de préférence inférieur à 7.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 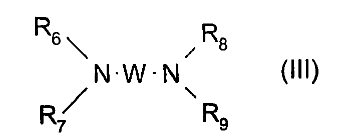 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

L'invention a aussi pour objet un procédé de coloration qui consiste à appliquer sur les fibres kératiniques une composition de coloration telle que définie précédemment pendant un temps suffisant pour obtenir la coloration désirée. Le temps de pose est généralement compris entre 1 à 60 minutes environ, de préférence 10 à 60 minutes environ. Après le temps de pose, les fibres kératiniques sont rincées laissant apparaître des fibres colorées.

La composition de l'invention peut de plus comprendre un agent oxydant.

Lorsque la composition de l'invention contient uniquement des colorants directs, cet agent oxydant permet d'obtenir une coloration éclaircissante, c'est-à-dire une décoloration et coloration simultanée des cheveux.

Lorsque la composition tinctoriale comprend une base d'oxydation et/ou un coupleur, il est nécessaire de mettre en contact cette composition avec un agent oxydant afin de former l'espèce colorée. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, cette composition étant appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Le procédé de coloration peut être mis en oeuvre à température ambiante ou à des températures plus élevées par exemple en utilisant un sèche cheveux, un casque de séchage, un fer à lisser, etc. Selon un mode de réalisation particulier, la température est comprise entre la température ambiante et 200°C, de préférence entre la température ambiante et 60° C.

Les exemples qui suivent servent à illustrer l'invention.

### EXEMPLES

### Exemple 1

La composition suivante a été préparée :

| | |
|---|---|
| Propylene glycol n-propyl ether | 20% |
| acide benzoïque | 0,2% |
| Disperse Red 13 | 0,2% |
| Eau | QSP 100 |

La composition est appliquée sur une mèche de cheveux naturels contenant 90% de cheveux blancs ainsi que sur une mèche de cheveux permanentés contenant 90% de cheveux blancs. Après 30 minutes à température ambiante, les mèches sont rincées et séchées. Les cheveux sont alors colorés en rouge.

### Exemple 2

La composition suivante a été préparée :

| | |
|---|---|
| éthanol | 10% |
| tripropylene glycol methyl ether | 10% |
| acide benzoïque | 2% |
| Disperse Red 13 | 0,3% |
| eau | QSP 100 |

La composition est appliquée sur une mèche de cheveux naturels contenant 90% de cheveux blancs ainsi que sur une mèche de cheveux permanentés contenant 90% de cheveux blancs. Après 30 minutes à température ambiante, les mèches sont rincées et séchées. Les mèches sont alors colorées en rouge.

## Revendications

1. Composition de coloration cosmétique comprenant dans un milieu de coloration cosmétique approprié, au moins un colorant direct hydrophobe dont le logP est supérieur à 2, le milieu approprié contenant au moins 40 % d'eau, en poids par rapport au poids total de la composition de coloration" et au moins un dérivé de propylène glycol de formule (I) suivante :
R₁(OC₃H₆)ₙOR₂ (I)
dans laquelle R₁ représente l'hydrogène, un radical alkyle en C₁-C₆ ou acyle C₂-C₆, R₂ représente un radical alkyle en C₁-C₆ ou aryle en C₆-C₃₀ et n variant de 1 à 6, le motif OC₃H₆ pouvant être linéaire ou ramifié.

2. Composition selon la revendication 1 dans laquelle au moins un des colorants directs hydrophobes présente un logP supérieur à 4.

3. Composition selon la revendication 1 ou 2 dans laquelle le colorant hydrophobe est choisi parmi
| **Colorant** | **Structure chimique** | **logP** |
|---|---|---|
| Disperse Red 13 | 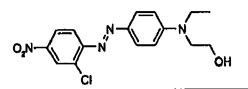 | 5.22 |
| Disperse Green 9 | 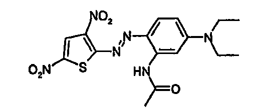 | 4.23 |
| Solvent Black 3 | 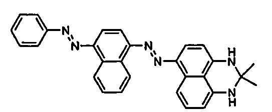 | 7.50 |
| Disperse Blue 148 | 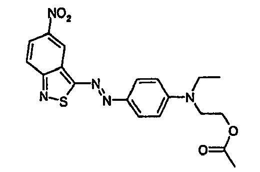 | 4.81 |
| Disperse Violet 63 | 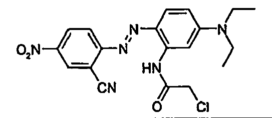 | 5.30 |
| Disperse Blue 60 | 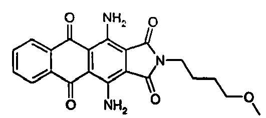 | 3.38 |
| Disperse Blue 14 | 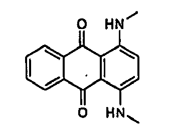 | 4.25 |
| Solvent Orange 15 | 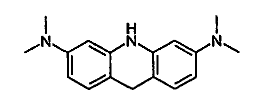 | 3.90 |
| Solvent Orange 7 | 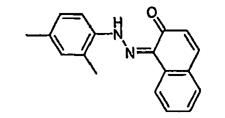 | 4.40 |
| Solvent Blue 14 | 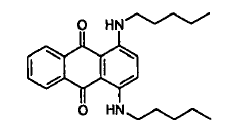 | 8.18 |
| Disperse Yellow 82 | 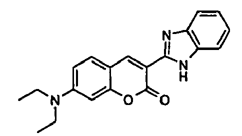 | 3.68 |

4. Composition selon l'une quelconque des revendications 1 à 3 dans laquelle le ou les colorants directs hydrophobes présentant un logP supérieur à 2 sont présents en quantité comprise entre 0,001 à 10 % en poids environ du poids total de la composition.

5. Composition selon l'une quelconque des revendications précédentes 1 à 4 dans laquelle le dérivé de propylène glycol de formule (I) est tel que n est compris entre 1 et 4, inclus et R1 représente un radical alkyle en C1-C6, de préférence C1-C4.

6. Composition selon la revendication 1 à 4 dans laquelle si R1 représente l'hydrogène alors n est supérieur à 1 et si n est égal à 1 alors R2 est un radical alkyle en C2-C6.

7. Composition selon l'une quelconque des revendications précédentes dans laquelle la quantité de dérivé de propylène glycol est comprise entre 0,1 et 40 % en poids du poids total de la composition, de préférence comprise entre 0,5 et 30 %, préférentiellement encore entre 1 et 20 %.

8. Composition selon l'une quelconque des revendications précédentes dans laquelle le dérivé de propylène glycol est choisi parmi
| | |
|---|---|
| Dipropylène glycol méthyl éther (Dowanol DPM) | CH₃O(C₃H₆O)₂H |
| Tripropylène glycol méthyl éther (Dowanol TPM) | CH₃O(C₃H₆O)₃H |
| Propylène glycol méthyl éther acétate (Dowanol PMA) | CH₃OC₃H₆OCOCH₃ |
| Dipropylène glycol méthyl éther acétate (Dowanol DPMA) | CH₃O(C₃H₆O)₂COCH₃ |
| Propylène glycol n-propyl éther (Dowanol PnP) | C₃H₇OCH₂CH(CH₃)OH |
| Propylène glycol n-butyl éther (Dowanol PnB) | C₄H₉OCH₂CH(CH₃)OH |
| Propylène glycol phényl éther (Dowanol PPh) | C₆H₅OC₃H₆OH |

9. Composition selon l'une quelconque des revendications 1 à 8 dans laquelle le milieu approprié à la coloration des fibres kératiniques comprend au moins 70%.

10. Composition selon l'une quelconque des revendications comprenant un ou plusieurs colorants directs additionnels choisis parmi les colorants directs nitrés benzéniques neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

11. Composition selon l'une quelconque des revendications précédentes comprenant une base d'oxydation choisie parmi les paraphénylènediamines, les bis-phénylalkylènediamines, les para-aminophénols, les ortho-aminophénols, les bases hétérocycliques et leurs sels d'addition.

12. Composition selon l'une quelconque des revendications précédentes comprenant un coupleur choisi parmi les métaphénylènediamines, les méta-aminophénols, les métadiphénols, les coupleurs naphtaléniques, les coupleurs hétérocycliques et leurs sels d'addition.

13. Composition selon la revendication 11 dans laquelle la quantité chacune des bases d'oxydation est comprise entre 0,001 à 10 % en poids environ du poids total de la composition tinctoriale.

14. Composition selon la revendication 12 dans laquelle la quantité de chacun des coupleurs est comprise entre 0,001 et 10 % en poids environ du poids total de la composition tinctoriale.

15. Composition selon l'une quelconque des revendications 1 à 14 comprenant un ou plusieurs adjuvants choisis parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, les agents épaississants associatifs polymères anioniques, cationiques, non ioniques et amphotères, les agents antioxydants, les agents de pénétration, les agents séquestrants, les parfums, les tampons, les agents dispersants, les agents de conditionnement, les agents filmogènes, les céramides, les agents conservateurs, les agents opacifiants.

16. Composition selon la revendication 15 dans laquelle les adjuvants sont présents en quantité comprise pour chacun d'eux entre 0,01 et 20 % en poids par rapport au poids de la composition.

17. Composition selon l'une quelconque des revendications précédentes comprenant de plus un agent oxydant.

18. Procédé de coloration des fibres kératiniques qui comprend l'application sur ces fibres d'une composition telle que définie selon l'une quelconque des revendications 1 à 17 pendant un temps suffisant pour obtenir la coloration désirée, suivi d'un rinçage.

19. Utilisation pour la coloration des fibres kératiniques d'une composition comprenant dans un milieu de coloration cosmétique approprié, au moins un colorant direct hydrophobe dont le logP est supérieur à 2, le milieu approprié contenant de l'eau, et au moins un dérivé de propylène glycol de formule (I) suivante :
R₁(OC₃H₆)ₙOR₂ (I)
dans laquelle R₁ représente l'hydrogène, un radical alkyle en C₁-C₆ ou acyle C₂-C₆, R₂ représente un radical alkyle en C₁-C₆ ou aryle en C₆-C₃₀ et n variant de 1 à 6, le motif OC₃H₆ pouvant être linéaire ou ramifié.

20. Utilisation selon la revendication 19 pour augmenter la ténacité de la coloration des fibres kératiniques.

21. Kit de coloration comprenant d'une part une composition comprenant une matière colorante et un dérivé de propylène glycol de formule (I) tels que définis aux revendications 1 à 16 et d'autre part une composition contenant un agent oxydant.

## Claims

1. Cosmetic dye composition comprising, in a suitable cosmetic dyeing medium, at least one hydrophobic direct dye whose logP is greater than 2, the suitable medium containing at least 40% of water, by weight relative to the total weight of the dye composition, and at least one propylene glycol derivative of formula (I) below:
R₁(OC₃H₆)ₙOR₂ (I)
in which R₁ represents hydrogen or a C₁-C₆ alkyl or C₂-C₆ acyl radical, R₂ represents a C₁-C₆ alkyl or C₆-C₃₀ aryl radical and n ranges from 1 to 6 inclusive, the OC₃H₆ unit possibly being linear or branched.

2. Composition according to Claim 1, in which at least one of the hydrophobic direct dyes has a logP of greater than 4.

3. Composition according to Claim 1 or 2, in which the hydrophobic dye is chosen from
| **Dye** | **Chemical structure** | **logP** |
|---|---|---|
| Disperse Red 13 | 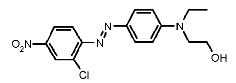 | 5.22 |
| Disperse Green 9 | 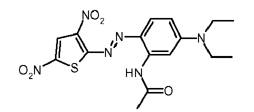 | 4.23 |
| Solvent Black 3 | 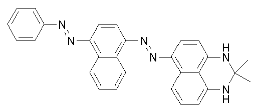 | 7.50 |
| Disperse Blue 148 | 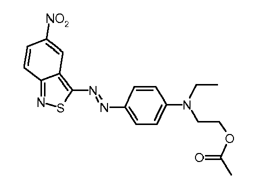 | 4.81 |
| Disperse Violet 63 | 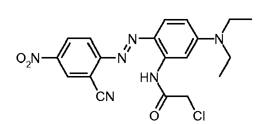 | 5.30 |
| Disperse Blue 60 | 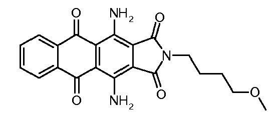 | 3.38 |
| Disperse Blue 14 | 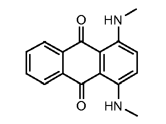 | 4.25 |
| Solvent Orange 15 | 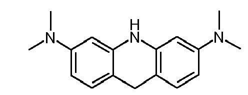 | 3.90 |
| Solvent Orange 7 | 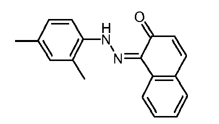 | 4.40 |
| Solvent Blue 14 | 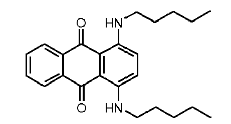 | 8.18 |
| Disperse Yellow 82 | 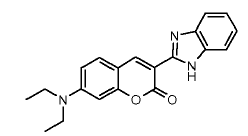 | 3.68 |

4. Composition according to any one of Claims 1 to 3, in which the hydrophobic direct dye(s) with a logP of greater than 2 is (are) present in an amount of between 0.001% and 10% by weight approximately relative to the total weight of the composition.

5. Composition according to any one of the preceding Claims 1 to 4, in which the propylene glycol derivative of formula (I) is such that n is between 1 and 4 inclusive and R₁ represents a C₁-C₆ and preferably C₁-C₄ alkyl radical.

6. Composition according to Claims 1 to 4, in which, if R₁ represents hydrogen, then n is greater than 1, and if n is equal to 1, then R₂ is a C₂-C₆ alkyl radical.

7. Composition according to any one of the preceding claims, in which the amount of propylene glycol derivative is between 0.1% and 40%, preferably between 0.5% and 30% and more preferentially between 1% and 20% by weight relative to the total weight of the composition.

8. Composition according to any one of the preceding claims, in which the propylene glycol derivative is chosen from
| | |
|---|---|
| Dipropylene glycol methyl ether (Dowanol DPM) | CH₃O(C₃H₆O)₂H |
| Tripropylene glycol methyl ether (Dowanol TPM) | CH₃O(C₃H6O)₃H |
| Propylene glycol methyl ether acetate (Dowanol PMA) | CH₃OC₃H₆OCOCH₃ |
| Dipropylene glycol methyl ether acetate (Dowanol DPMA) | CH₃O(C₃H₆O)₂COCH₃ |
| Propylene glycol n-propyl ether (Dowanol PnP) | C₃H₇OCH₂CH(CH₃) OH |
| Propylene glycol n-butyl ether (Dowanol PnB) | C₄H₉OCH₂CH(CH₃)OH |
| Propylene glycol phenyl ether (Dowanol PPh) | C₆H₅OC₃H₆OH |

9. Composition according to any one of Claims 1 to 8, in which the medium that is suitable for dyeing keratin fibres comprises at least 70% by weight of water relative to the total weight of the dye composition.

10. Composition according to any one of the preceding claims, comprising one or more additional direct dyes chosen from neutral, acidic or cationic nitrobenzene direct dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

11. Composition according to any one of the preceding claims, comprising an oxidation base chosen from para-phenylenediamines, bis(phenyl)alkylenediamines, para-aminophenols, ortho-aminophenols and heterocyclic bases, and the addition salts thereof.

12. Composition according to any one of the preceding claims, comprising a coupler chosen from meta-phenylenediamines, meta-aminophenols, meta-diphenols, naphthalene-based couplers and heterocyclic couplers, and the addition salts thereof.

13. Composition according to Claim 11, in which the amount of each of the oxidation bases is between 0.001% and 10% by weight approximately relative to the total weight of the dye composition.

14. Composition according to Claim 12, in which the amount of each of the couplers is between 0.001% and 10% by weight approximately relative to the total weight of the dye composition.

15. Composition according to any one of Claims 1 to 14, comprising one or more adjuvants chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, anionic, cationic, nonionic and amphoteric polymeric associative thickeners, antioxidants, penetrants, sequestrants, fragrances, buffers, dispersants, conditioning agents, film-forming agents, ceramides, preserving agents and opacifiers.

16. Composition according to Claim 15, in which the adjuvants are present in an amount for each of them of between 0.01% and 20% by weight relative to the weight of the composition.

17. Composition according to any one of the preceding claims, also comprising an oxidizing agent.

18. Process for dyeing keratin fibres, which comprises the application to these fibres of a composition as defined according to any one of Claims 1 to 17, for a time that is sufficient to obtain the desired coloration, followed by rinsing.

19. Use, for the dyeing of keratin fibres, of a composition comprising, in a suitable cosmetic dyeing medium, at least one hydrophobic direct dye whose logP is greater than 2, the suitable medium containing water, and at least one propylene glycol derivative of formula (I) below:
R₁(OC₃H₆)ₙOR₂ (I)
in which R₁ represents hydrogen or a C₁-C₆ alkyl or C₂-C₆ acyl radical, R₂ represents a C₁-C₆ alkyl or C₆-C₃₀ aryl radical and n ranges from 1 to 6 inclusive, the OC₃H₆ unit possibly being linear or branched.

20. Use according to Claim 19, for increasing the coloration fastness of keratin fibres.

21. Dyeing kit comprising, firstly, a composition comprising a dyestuff and a propylene glycol derivative of formula (I) as defined in Claims 1 to 16, and, secondly, a composition containing an oxidizing agent.

## Patentansprüche

1. Kosmetische Färbezusammensetzung, die in einem geeigneten kosmetischen Färbemedium mindestens einen hydrophoben Direktfarbstoff mit einem logP-Wert von mehr als 2 umfaßt, wobei das geeignete Medium mindestens 40 Gew.-% Wasser, bezogen auf das Gesamtgewicht der Färbezusammensetzung, und mindestens ein Propylenglykolderivat der folgenden Formel (I) :
R₁(OC₃H₆)ₙOR₂ (I)
worin R₁ für Wasserstoff, einen C₁-C₆-Alkyl- oder C₂-C₆-Acylrest steht, R₂ für einen C₁-C₆-Alkyl oder C₆-C₃₀-Arylrest steht und n im Bereich von 1 bis 6 liegt, wobei die OC₃H₆-Einheit linear oder verzweigt sein kann, enthält.

2. Zusammensetzung nach Anspruch 1, in der mindestens einer der hydrophoben Direktfarbstoffe einen logP-Wert von mehr als 4 aufweist.

3. Zusammensetzung nach Anspruch 1 oder 2, in der der hydrophobe Farbstoff unter
| **Farbstoff** | **Chemische Struktur** | **logP** |
|---|---|---|
| Disperse Red 13 | 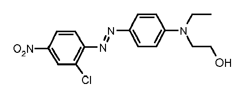 | 5,22 |
| Disperse Green 9 | 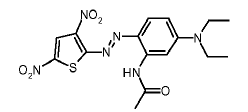 | 4,23 |
| Solvent Black 3 | 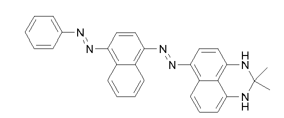 | 7,50 |
| Disperse Blue 148 | 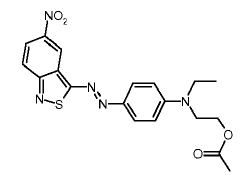 | 4,81 |
| Disperse Violet 63 | 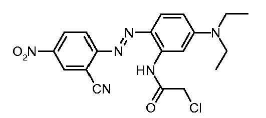 | 5,30 |
| Disperse Blue 60 | 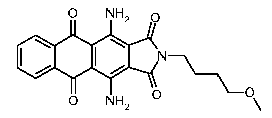 | 3,38 |
| Disperse Blue 14 | 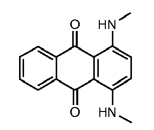 | 4,25 |
| Solvent Orange 15 | 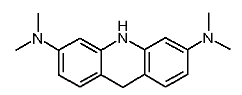 | 3, 90 |
| Solvent Orange 7 | 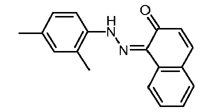 | 4,40 |
| Solvent Blue 14 | 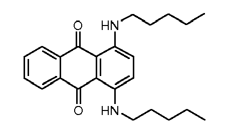 | 8,18 |
| Disperse Yellow 82 | 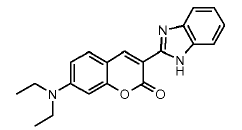 | 3, 68 |
ausgewählt ist.

4. Zusammensetzung nach einem der Ansprüche 1 bis 3, in der der bzw. die hydrophoben Direktfarbstoffe mit einem logP-Wert von mehr als 2 in einer Menge zwischen ungefähr 0,001 und 10 Gew.-% des Gesamtgewichts der Zusammensetzung vorliegen.

5. Zusammensetzung nach einem der vorhergehenden Ansprüche 1 bis 4, in der das Propylenglykolderivat der Formel (I) so beschaffen ist, daß n zwischen 1 und 4 einschließlich liegt und R1 für einen C₁-C₆-Alkylrest, vorzugsweise C₁-C₄-Alkylrest, steht.

6. Zusammensetzung nach Anspruch 1 bis 4, in der dann, wenn R1 für Wasserstoff steht, n größer 1 ist und dann, wenn n gleich 1 ist, R2 ein C2-C6-Alkylrest ist.

7. Zusammensetzung nach einem der vorhergehenden Ansprüche, in der die Menge des Propylenglykolderivats zwischen 0,1 und 40 Gew.-% des Gesamtgewichts der Zusammensetzung, vorzugsweise zwischen 0,5 und 30 Gew.-%, weiter bevorzugt zwischen 1 und 20 Gew.-%, liegt.

8. Zusammensetzung nach einem der vorhergehenden Ansprüche, in der das Propylenglykolderivat unter
| | |
|---|---|
| Dipropylenglykolmethylether (Dowanol DPM) | CH₃O(C₃H₆O)₂H |
| Tripropylenglykolmethylether (Dowanol TPM) | CH₃O(C₃H₆O)₃H |
| Propylenglykolmethyletheracetat (Dowanol PMA) | CH₃OC₃H₆OCOCH₃ |
| Dipropylenglykolmethyletheracetat (Dowanol DPMA) | CH₃O(C₃H₆O)₂COCH₃ |
| Propylenglykol-n-propylether (Dowanol PnP) | C₃H₇OCH₂CH (CH₃)OH |
| Propylenglykol-n-butylether (Dowanol PnB) | C₄H₉OCH₂CH (CH₃)OH |
| Propyleneglykolphenylether (Dowanol PPh) | C₆H₅OC₃H₆OH |
ausgewählt ist.

9. Zusammensetzung nach einem der Ansprüche 1 bis 8, in der das zum Färben von Keratinfasern geeignete Medium mindestens 70% umfaßt.

10. Zusammensetzung nach einem der Ansprüche, die ein oder mehrere zusätzliche Direktfarbstoffe umfaßt, die unter neutralen, sauren oder kationischen Nitrobenzol-Direktfarbstoffen, neutralen, sauren oder kationischen Azo-Direktfarbstoffen, neutralen, sauren oder kationischen Chinon- und insbesondere Anthrachinon-Direktfarbstoffen, Azin-Direktfarbstoffen, Triarylmethan-Direktfarbstoffen, Indoamin-Direktfarbstoffen und natürlichen Direktfarbstoffen ausgewählt sind.

11. Zusammensetzung nach einem der vorhergehenden Ansprüche, die eine Oxidationsbase umfaßt, die unter para-Phenylendiaminen, Bis(phenyl)alkylen-diaminen, para-Aminophenolen, ortho-Aminophenolen, heterocyclischen Basen und Additionssalzen davon ausgewählt ist.

12. Zusammensetzung nach einem der vorhergehenden Ansprüche, die einen Kuppler umfaßt, der unter meta-Phenylendiaminen, meta-Aminophenolen, meta-Diphenolen, Naphthalin-Kupplern, heterocyclischen Kupplern und Additionssalzen davon ausgewählt ist.

13. Zusammensetzung nach Anspruch 11, in der die Menge jeder der Oxidationsbasen zwischen ungefähr 0,001 und 10 Gew.-% des Gesamtgewichts der Färbezusammensetzung liegt.

14. Zusammensetzung nach Anspruch 12, in der die Menge jedes der Kuppler zwischen ungefähr 0,001 und 10 Gew.-% des Gesamtgewichts der Färbezusammensetzung liegt.

15. Zusammensetzung nach einem der Ansprüche 1 bis 14, die einen oder mehrere Hilfsstoffe umfaßt, die unter anionischen, kationischen, nichtionischen, amphoteren oder zwitterionischen Tensiden oder Mischungen davon, anionischen, kationischen, nichtionischen, amphoteren oder zwitterionischen Polymeren oder Mischungen davon, anorganischen oder organischen Verdickungsmitteln, anionischen, kationischen, nichtionischen und amphoteren polymeren Assoziativverdickern, Antioxidantien, Penetriermitteln, Sequestriermitteln, Parfümen, Puffern, Dispergiermitteln, Konditionierungsmitteln, Filmbildnern, Ceramiden, Konservierungsmitteln und Trübungsmitteln ausgewählt sind.

16. Zusammensetzung nach Anspruch 15, in der die Menge der Hilfsstoffe jeweils zwischen 0,01 und 20 Gew.-%, bezogen auf das Gewicht der Zusammensetzung, liegt.

17. Zusammensetzung nach einem der vorhergehenden Ansprüche, die außerdem ein Oxidationsmittel umfaßt.

18. Verfahren zum Färben von Keratinfasern, bei dem man auf diese Fasern eine Zusammensetzung gemäß einem der Ansprüche 1 bis 17 über einen zur Erzielung der gewünschten Färbung ausreichenden Zeitraum aufbringt und danach spült.

19. Verwendung einer Zusammensetzung, die in einem geeigneten kosmetischen Färbemedium mindestens einen hydrophoben Direktfarbstoff mit einem logP-Wert von mehr als 2 umfaßt, wobei das geeignete Medium Wasser und mindestens ein Propylenglykolderivat der folgenden Formel (I):
R₁(OC₃H₆)ₙOR₂ (I)
worin R₁ für Wasserstoff, einen C₁-C₆-Alkyl- oder C₂-C₆-Acylrest steht, R₂ für einen C₁-C₆-Alkyl oder C₆-C₃₀-Arylrest steht und n im Bereich von 1 bis 6 liegt, wobei die OC₃H₆-Einheit linear oder verzweigt sein kann, enthält,
zum Färben von Keratinfasern.

20. Verwendung nach Anspruch 19 zur Erhöhung der Beständigkeit von Färbungen von Keratinfasern.

21. Färbekit, das zum einen eine Zusammensetzung, die einen Farbstoff und ein Propylenglykolderivat der Formel (I) gemäß den Ansprüchen 1 bis 16 umfaßt, und zum anderen eine Zusammensetzung, die ein Oxidationsmittel enthält, umfaßt.
